(19) Europäisches Patentamt
European Patent Office
Office européen des brevets

(11) Publication number: 0 363 589 A2

(12) # EUROPEAN PATENT APPLICATION

(21) Application number: 89114365.3

(22) Date of filing: 03.08.89

(51) Int. Cl.5: C07K 7/26 , C07K 1/00 , A61K 37/43

The title of the invention has been amended (Guidelines for Examination in the EPO, A-III, 7.3).

(30) Priority: 18.08.88 US 233690

(43) Date of publication of application: 18.04.90 Bulletin 90/16

(84) Designated Contracting States: AT BE CH DE ES FR GB GR IT LI LU NL SE

(71) Applicant: SYNTEX (U.S.A.) INC.
3401 Hillview Avenue
Palo Alto California 94303(US)

(72) Inventor: Nestor, John J.
20937 Fairwoods Court
Cupertino California 95014(US)
Inventor: Vickery, Brian H.
345 Forest Avenue No.402
Palo Alto California 94301(US)

(74) Representative: Barz, Peter, Dr. et al
Patentanwälte Dipl.-Ing. G. Dannenberg Dr. P. Weinhold, Dr. D. Gudel Dipl.-Ing. S. Schubert, Dr. P. Barz Siegfriedstrasse 8
D-8000 München 40(DE)

(54) **Somatostatin analogues.**

(57) Pharmaceutical compounds having somatostatin-like activity which compounds comprise a peptide analog of somatostatin linked to a cationic anchor.

EP 0 363 589 A2

## PHARMACEUTICAL COMPOUNDS

This invention relates to pharmaceutical compounds exhibiting somatostatin-like activity, the method of preparation of such compounds and their use in the regulation of hormone secretion in the body. In particular, the pharmaceutical compounds are somatostatin analogs comprising a cationic anchor connected to a somatostatin-like cyclic peptide.

Somatostatin is a naturally occurring compound of the formula:

Ala-Gly-Cys-Lys-Asn-Phe-Phe-Trp-Lys-Thr-Phe-Thr-Ser-Cys-OH

It is well known in the art that somatostatin has powerful biological effects as an inhibitory hormone in many tissues. For example, somatostatin blocks secretion of growth hormone from the pituitary, secretion of gastric acid and pepsin from the stomach lining, and release of both glucagon and insulin from the pancreatic islets. Somatostatin also acts to inhibit the absorption of glucose, amino acids and fat from the gastrointestinal (GI) tract as well as inhibit GI motility.

Because the naturally occurring somatostatin has a very short duration of action in the body, attempts have been made to create compounds that are longer acting and more potent than somatostatin, while retaining the characteristics of somatostatin, i.e. "somatostatin-like" compounds. Modifications of the amino acid sequence of somatostatin have, in some instances, resulted in an increase in potency, duration of action, relative specificity and other desirable traits.

Somatostatin-like compounds for use as growth hormone secretion inhibitors are described in U.S. Patent No. 4,291,022 issued September 22, 1981. The compounds are derivatives of naturally occuring somatostatin.

Somatostain analogs comprising a cyclic hexapeptide in which seven of the ring amino acids of somatostatin have been deleted are described in U.S. Patent No. 4,310,518 issued January 12, 1982. The compounds are shown to inhibit the release of growth hormone, insulin and glucagon.

Cyclic hexapeptides and bridged cyclic somatostatin analogs for use in the treatment of immediate hypersensitivity are disclosed in European Patent Application 0 222 578, assigned to Merck & Co. Inc. The use of somatostatin analogs for the treatment of dermatological diseases is disclosed in European Patent Application 0 175 644, assigned to Sandoz AG.

Cai, et. al., Proc. Natl. Acad. Sci. USA 83: 1896 (1986) describe an octapeptide analog of somatostatin that shows prolonged duration of action in the inhibition of growth hormone release as compared to somatostatin. Data was also presented showing some inhibition of tumor growth.

Bauer, et. al., Life Sciences 31:1133 (1982) describe specific analogs of somatostatin exhibiting prolonged action and a three fold increase in potency over somatostatin in inhibiting the secretion of growth hormone. The octapeptides described were specifically modified at the cysteine residues of the molecule and were shown to have particular value in the treatment of acromegaly.

United States Patent No. 4,342,671 issued August 3, 1982 describes somatostatin analogs with selective modifications at certain positions within the peptide. The modified somatostatin analogs were shown to have the same activity as the native molecule.

Kurashima, et.al., Life Sciences 41:1011 (1987) describe the effects of highly potent octapeptide analogs of somatostatin on growth hormone, insulin and glucagon release.

Redding and Schally, Proc. Natl. Acad. Sci. USA 80:1078 (1983) describe the use of somotostatin analogs in the inhibition of growth of transplantable rat chondrosarcoma.

Moreau, et.al., Life Sciences 40:419 (1987) provide a recent mini-review of the current work being conducted in the area of somatostatin and somatostatin analogs. Therapeutic advances and ongoing pharmacological studies are discussed.

In the instant invention, the addition of a cationic anchor caused an increased duration of action and greatly increased specificity of action. The cationic anchor of the instant invention is of the $N^{\omega}$alkyl basic amino acid class, both natural and non-natural. The use of non-naturally occurring amino acids of the $N^{\omega}$alkyl amino acid class incorporated into antagnostic analogues of luteinizing hormone-releasing hormone (LH-RH) as a means to stabilize a hypothesized phospholipid membrane interaction has recently been described, Nestor, et.al., J. Med. Chem., 31:65 (1988). However, the incorporation of cationic anchor in a somatostatin analog structure has not been disclosed before. There is scientific evidence suggesting that

the specificity of ligand-receptor interaction may involve ligand phospholipid membrane interaction (Schwyzer, Biochemistry 25:6335 (1986). The addition of the cationic anchors described herein to somatostatin-like cyclic peptides has resulting in greatly increased potency, duration of action and selectivity of action.

The key aspect of the compounds of this invention is the combination of a cationic anchor attached to a somatostatin-like cyclic peptide, either directly or through a spacer. As a result of the attachment of the cationic anchor, the compounds of the invention show enhanced activity, duration of action, and specificity.

One embodiment of the invention is a compound of the formula

X-Cys-D-D-Trp-E-F-Cys-Y (I)

and the pharmaceutically acceptable salts thereof wherein,

X is an N-terminus anchor;

Y is a C-terminus anchor, G-I or alcohol of G-I; wherein at least one of X and Y is a cationic anchor;

D is Phe, Tyr, pF-Phe, pCl-Phe;

E is Lys or Lys($R_1$) wherein $R_1$ is a lower alkyl or fluoroalkyl of from 1 to 8 carbon atoms;

F is Thr, Val, Ser:

G is D or L configuration of Thr, Phe, or Nal(2); and

I is -OH, -NHR$_2$ where $R_2$ is H or a substituent selected from $R_1$ above.

Another embodiment of the invention is a somatostatin-like compound of the formula:

X-Cys-Lys-Asn-Phe-D-DTrp-E-F-Phe-Thr-Ser-Cys-Y

(II)

and the pharmaceutically acceptable salts thereof wherein,

X is an N-terminus anchor;

Y is a C-terminus anchor, G-I or alcohol of G-I; wherein at least one of X and Y is a cationic anchor;

D is Phe, Tyr, pF-Phe, pCl-Phe;

E is Lys or Lys($R_1$) where $R_1$ is a lower alkyl or fluoroalkyl of from 1 to 8 carbon atoms;

F is Thr, Val, Ser:

G is D or L configuration of Thr, Phe, Nal(2), or $(Gly)_m$, and m is 0 to 3; and

I is -OH, -NHR$_2$ where $R_2$ is H or a substituent selected from $R_1$ above.

In yet another embodiment of the invention a method for preparing the compounds of the invention and pharmaceutically acceptable salts thereof is described. The method comprises removing protecting groups and, optionally, covalently bound solid support from a protected polypeptide to afford a compound, wherein the compound comprises: (a) a cyclical peptide having somatostatin-like activity, and (b) a cationic anchor attached to one or both of the cysteine residues of the peptide.

In particular, the methods for preparing a compound of the formula:

X-Cys-D-D-Trp-E-F-Cys-Y (I)

and the pharmaceutically acceptable salts thereof,
wherein, X, Y, D, E and F are as defined above; comprise:

(a) removing protecting groups from a compound of the formula:

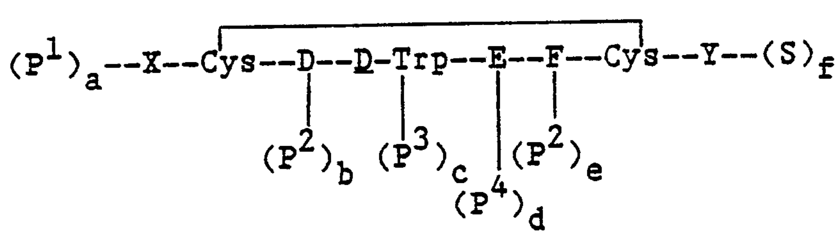

wherein,
X, Y, D, E and F are as defined above;
$P^1$, $P^2$, $P^3$ and $P^4$ are protecting groups wherein $P^1$ is an α-amino protecting group, $P^2$ is a hydroxyl protecting group, $P^3$ is an $N^{IN}$-protecting group and $P^4$ is an ε-amino protecting group;
S is a carboxyl protecting group; and
each of a, b, c, d, e and f is an integer equal to 0 or 1, provided that all of a to f cannot be 0; or

(b) simultaneously removing protecting groups and a polymeric support from a compound of the formula:

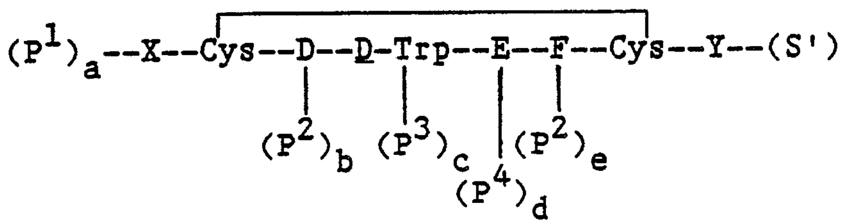

wherein,
X, Y, D, E, F, $P^1$, $P^2$, $P^3$ and $P^4$ are as defined in paragraph (a) above;
each of a, b, c, d and e is an integer equal to 0 or 1, provided that all of a to e cannot be 0; and
S′ is a polymeric support; or

(c) removing a polymeric support from a compound of the formula:

X--Cys--D--D-Trp--E--F--Cys--Y--(S')

wherein,
X, Y, D, E, F and S′ are as defined in paragraph (b) above; or

(d) oxidizing a compound of the formula:

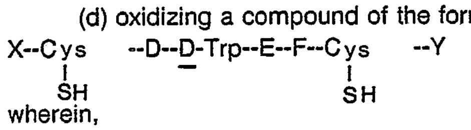

wherein,
X, Y, D, E and F are as defined in paragraph (a) above; or

(d) converting a compound of the formula I to a pharmaceutically acceptable salt; or

(e) converting a salt of the compound of the formula I to another pharmaceutically acceptable salt; or

(f) converting a salt of the compound of the formula I to the free peptide; and the methods for preparing a compound of the formula:

X-Cys-Lys-Asn-Phe-D-D-Trp-E-F-Phe-Thr-Ser-Cys-Y (II)

wherein
X, Y, D, E and F are as defined above, and the pharmaceutically acceptable salts thereof, comprise:

(a) removing protecting groups from a compound of the formula:

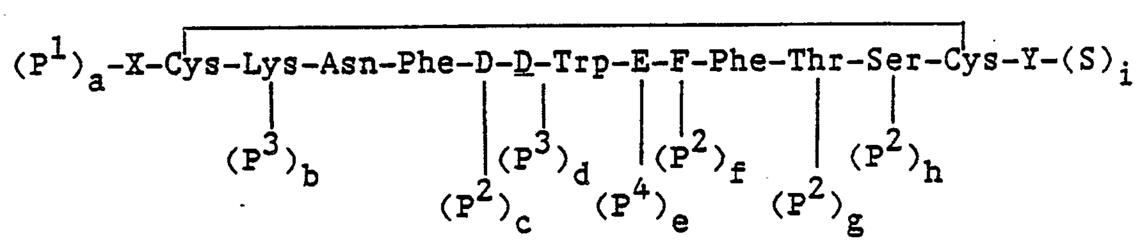

wherein,
X, Y, D, E and F are as defined above;

$P^1$, $P^2$, $P^3$ and $P^4$ are protecting groups wherein $P^1$ is an α-amino protecting group, $P^2$ is a hydroxyl protecting group, $P^3$ is an $N^{IN}$-protecting group and $P^4$ is an ε-amino protecting group;
S is a carboxyl protecting group; and
each of a, b, c, d, e, f, g, h and i is an integer equal to 0 or 1, provided that all of a to i cannot be 0; or

(b) simultaneously removing protecting groups and a polymeric support from a compound of the formula:

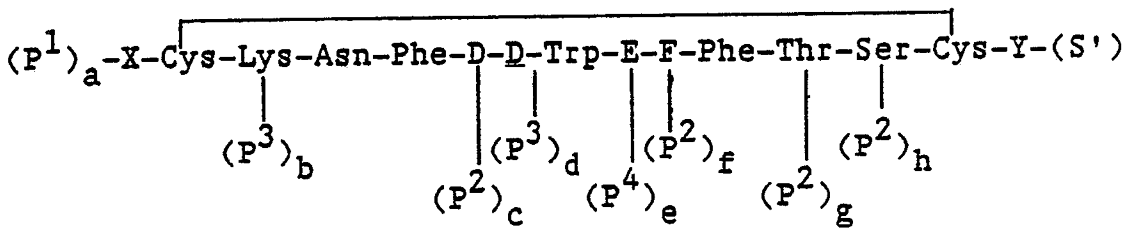

wherein,
X, Y, D, E, F, $P^1$, $P^2$, $P^3$ and $P^4$ are as defined in paragraph (a) above;
each of a, b, c, d, e, f, g and h is an integer equal to 0 or 1, provided that all of a to h cannot be 0; and
S′ is a polymeric support; or

(c) removing a polymeric support from a compound of the formula:

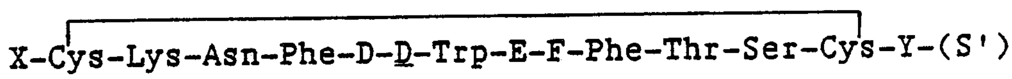

wherein,
X, Y, D, E, F and S′ are as defined in paragraph (b) above; or

(d) oxidizing a compound of the formula:

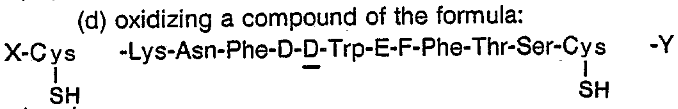

wherein,
X, Y, D, E and F are as defined in paragraph (a) above; or

(d) converting a compound of the formula II to a pharmaceutically acceptable salt; or

(e) converting a salt of the compound of the formula II to another pharmaceutically acceptable salt; or

(f) converting a salt of the compound of the formula II to the free peptide.

In yet another embodiment of the invention a composition for effective treatment of an individual is described wherein the pharmaceutical composition comprises an effective amount of a compound of the invention, optionally with a recognized pharmaceutical carrier.

## Abbreviations and Definitions

As set forth above, and for convenience in describing this invention, the conventional abbreviations for the various common amino acids are used as generally accepted in the peptide art as recommended by the IUPAC-IUB Commission on Biochemical Nomenclature, Biochem. J. 219:345 (1984). All peptide sequences mentioned herein are written according to the generally accepted convention whereby the N-terminal amino acid is on the left and the C-terminal amino acid is on the right.

The abbreviations herein represent L-amino acids unless otherwise designated as D- or D,L-. Certain amino acids, both natural or non-natural, are achiral, e.g. glycine.

Non-naturally occurring amino acids are designated as the appropriate amino acid with the substituent in pararenthesis. For example,
Lys($R_1$) is lysine with an $R_1$ substituent on the ω-amine, and
Arg($R_1$,$R_2$) and hArg($R_1$,$R_2$) are arginine and homo-arginine, respectively, with an $R_1$ substituent on the ω-nitrogen and an $R_2$ substituent on the ω′-nitrogen of the guanidino moiety.

Specific abbreviations of non-naturally occurring amino acids will be useful in describing the invention. Representative non-naturally occuring amino acids which can be employed include the following:

| Amino acid residue | Abbreviation |
|---|---|
| 3-(2-naphthyl)-alanyl | Nal(2) |
| 3-(p-fluorophenyl)-alanyl | pF-Phe |
| 3-(p-chlorophenyl)-alanyl | pCl-Phe |
| *N,N'-guanidino-dimethyl-homoarginyl | Dmh, or $hArg(Me)_2$ |
| *N,N'-guanidino-(diethyl)-homoarginyl | Deh, or $hArg(Et)_2$ |
| *N,N'-guanidino-(dipropyl)-homoarginyl | Dph, or $hArg(Pr)_2$ |
| *N,N'-guanidino-(diisopropyl)-homoarginyl | Dih, or Arg $(iPr)_2$ |

| | |
|---|---|
| *N,N'-guanidino-(dihexyl)-homoarginyl | Dhh, or hArg(hexyl)$_2$ |
| *N,N'-guanidino-(ethano)-homoarginyl | Eha or hArg($CH_2$)$_2$ |
| *N,N'-guanidino-(propano)-homoarginyl | Pha, or hArg($CH_2$)$_3$ |
| *N,N'-guanidino-bis-(2,2,2-trifluoroethyl)-homoarginyl | Bth, or hArg($CH_2CF_3$)$_2$ |
| *N-guanidino-(ethyl)-homoarginyl | Meh, or hArg(Et) |
| *N-guanidino-(propyl)-homoarginyl | Prh, or hArg(Pr) |
| *N-guanidino-(isopropyl)-homoarginyl | Iph, or hArg(iPr) |
| *N-guanidino-(butyl)-homoarginyl | Mbh, or hArg(Bu) |
| *N,N'-guanidino-(dicyclohexyl)-homoarginyl | Dch, or hArg(cyclohexyl)$_2$ |
| *N-guanidino-(heptyl)-homoarginyl | Hha, or hArg(heptyl) |
| *N-guanidino-(ethyl)-arginyl | Mea, or Arg(Et) |
| *N-guanidino-(hexyl,methyl)-arginyl | Hmh, or hArg(hexyl,methyl) |
| *N-guanidino-(butyl,methyl)-arginyl | Bmh, or hArg(butyl,methyl) |
| *$N^{\varepsilon}$-isopropyl-lysine | Ipl, or Lys(iPr) |
| *N,N'-guanidino-(diisopropyl)-arginyl | Dia, or Arg(iPr)$_2$ |
| *N,N'-guanidino-(dicyclohexyl)-arginyl | Dca, or Arg(cyclohexyl)$_2$ |

| | |
|---|---|
| *N,N'-guanidino-bis(3,3,3,2,2-pentafluoropropyl)homoarginyl | Bph |
| *N,N'-guanidino-(3,3,3,3,3-pentafluoropropyl)homoarginyl | Fph |
| *$N^G$-ethyl,$N^{G'}$-(2,2,2-trifluoroethyl)homoarginyl | Efh |
| *N,N'-guanidino-(diethyl)arginyl | Dea |
| *N,N'-guanidino-bis(2,2,2-trifluoroethyl)-arginyl | Bta |

The asterisked non-naturally occuring amino acids can function as cationic anchors in accordance with this invention.

The non-naturally occuring amino acids described herein are prepared by methods well known to those skilled in the art and may be used in either solution phase or solid phase peptide synthesis procedures. (See, for example, Nestor, et.al., supra (1988)).

As used herein, the term "pharmaceutically acceptable salts" refers to salts that retain the desired biological activity of the parent compound and do not impart any undesired toxicological effects. Examples of such salts are (a) acid addition salts formed with inorganic acids, for example hydrochloric acid, hydrobromic acid, sulfuric acid, phosphoric acid, nitric acid and the like; and salts formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, pamoic acid, alginic acid, polyglutamic acid, naphthalenesulfonic acids, naphthalenedisulfonic acids, polygalacturonic acid; (b) base addition salts formed with polyvalent metal cations such as zinc, calcium, bismuth, barium, magnesium, aluminum, copper, cobalt, nickel, cadmium, and the like; or with an organic cation formed from N,N'-dibenzylethylenediamine or ethylenediamine; or (c) combinations, of (a) and (b), e.g., a zinc tannate salt and the like.

The term "alkyl" refers to a straight or branched chain saturated hydrocarbon radical having from 1 to 8 carbon atoms. Examples of such alkyl groups with the corresponding abbreviation included in parenthesis, include but are not limited to methyl (Me), ethyl (Et), n-propyl (Pr), isopropyl (iPr), n-butyl (Bu), isobutyl (iBu), sec-butyl (s-Bu), tert-butyl (t-Bu), n-pentyl (Pe), n-hexyl (He) or branched 5 to 8 carbon membered radicals, and the like.

Fluoroalkyl refers to lower alkyl substituted with 1 to 5 fluorine atoms, for example $CF_3CH_2$-, $CF_3$-, $CF_3CF_2CH_2$-, and the like.

The abbreviation "N-Ac" refers specifically to the N-acetyl protecting group, i.e., an acetyl group attached to a terminal amino acid residue on the amine nitrogen, in conformance with generally acceptable nomenclature.

Acyl refers to the organic radical derived from an organic acid by the removal of the hydroxyl group. Generally, the acyl is attached to a terminal amino acid residue on the amine nitrogen.

An anchor refers to a biologically acceptable, covalently-bound, amino acid moiety that is attached to one of the Cys groups depicted in the compounds of formulae I and II. Amino acids moieties are derived from both naturally occuring amino acids, such as alanine, arginine, asparagine, aspartic acid, cysteine, glutamine, glutamic acid, glycine, histidine, isoleucine, lysine, methionine, phenylalanine, proline, serine, threonine, tryptophan, tyrosine, valine, and the like, and non-naturally occuring amino acids such as homocysteine, ornithine, homoarginine, β-alanine, the non-naturally occuring amino acids listed hereinabove, and the like. A cationic anchor will have a positively charged function, e.g. of the $N^{\omega}$-alkyl-basic amino acid class, which may be attached to the peptide either directly or through a spacer. Examples of $N^{\omega}$alkyl-basic amino acids include arginine, homoarginine, lysine, ornithine and the asterisked non-naturally occuring amino acids listed hereinabove. When the cationic anchor is attached to the N-terminus, a Thr-type amino acid residue, a hydrophobic amino acid residue or another cationic anchor may be attached to the C-terminus. Examples of hydrophobic amino acid residues include glycine, alanine, valine, leucine, isoleucine, tryptophan, phenylalanine, naphthylalanine, halo-substituted phenylalanine, and the like. When the cationic anchor is attached to the C-terminus, a hydrophobic residue or another cationic anchor may be attached to the N-terminus.

A somatostatin-like cyclic peptide is a cyclic peptidyl moiety having somatostatin receptor binding or

somatostatin-like biological activity.

An alcohol of G-I refers to the alcohol derived from the amino acid moiety which G-I represents. For example, if G-I is Thr-OH, i.e.

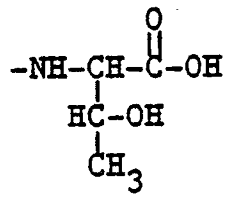

,

the alcohol would be

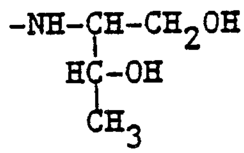

Preferred Embodiments

One preferred embodiment of the invention is a compound of formula I or II wherein X is a cationic anchor of the general formula:

$A\text{-}(B)_m\text{-}(C)_n$

where A is acyl or H;

B is hArg($R_1$,$R_2$), Arg($R_1$,$R_2$) or Lys($R_1$), where $R_1$ is lower alkyl or fluoroalkyl of from 1 to 8 carbon atoms and $R_2$ is H or a substituent selected from $R_1$ or other ω-alkyl-basic amino acids;

m is an integer from 1 to 3;

C is a spacer such as Gly, Ala, β-Ala; and

n is an integer from 0 to 5.

Compounds that are particularly useful are those where n is 0 or 1.

The cyclic somatostatin-like peptide with its bound cationic anchor may be either the full dodecapeptide disulfide ring or the cyclic hexapeptide and ring-substituted analogs thereof. In a most preferred practice of the invention the compound is

Ac-D-hArg$(CH_2CF_3)_2$-Gly-Cys-Phe-D-Trp-Lys-Thr-Cys-Thr-$NH_2$ or

Ac-D-hArg$(CH_2CF_3)_2$-D-hArg$(CH_2CF_3)_2$-Gly-Cys-Phe-D-Trp-Lys-Thr-Cys-Thr-$NH_2$ or

Ac-L-hArg$(CH_2CF_3)_2$-Gly-Cys-Phe-D-Trp-Lys-Thr-Cys-Thr-$NH_2$ or

Ac-D-hArg$(Et_2)$-Gly-Cys-Phe-D-Trp-Lys-Thr-Cys-Thr-$NH_2$ or

Ac-D-hArg$(Et_2)$-D-hArg$(Et_2)$-Gly-Cys-Phe-D-Trp-Lys-Thr-Cys-Thr-$NH_2$ or

Ac-D-hArg$(Et_2)$-Gly-Cys-Phe-D-Trp-Lys(Me)-Thr-Cys-Thr-NHEt

and the pharmaceutically acceptable salts thereof.

Other preferred compounds of the invention include:

Ac-hArg($Et_2$)-Gly-Cys-Phe-D-Trp-Lys-Thr-Cys-Thr-$NH_2$;

Ac-D-hArg($Et_2$)-Gly-Cys-Phe-D-Trp-Lys-Thr-Cys-Thr-$NH_2$;

Ac-D-hArg(Bu)-Gly-Cys-Phe-D-Trp-Lys-Thr-Cys-Thr-$NH_2$;

Ac-L-hArg($Et_2$)-Cys-Phe-D-Trp-Lys-Thr-Cys-Thr-$NH_2$;

Ac-D-hArg($Et_2$)-Cys-Phe-D-Trp-Lys-Thr-Cys-Thr-$NH_2$;

Ac-D-hArg($CH_2CF_3$)$_2$-Cys-Phe-D-Trp-Lys-Thr-Cys-Thr-$NH_2$;

Ac-hArg($CH_3$,hexyl)-Gly-Cys-Phe-D-Trp-Lys-Thr-Cys-Thr-$NH_2$;

H-hArg($hexyl_2$)-Gly-Cys-Phe-D-Trp-Lys-Thr-Cys-Thr-$NH_2$;

Ac-D-hArg($Et_2$)-Gly-Cys-Phe-D-Trp-Lys-Thr-Cys-Thr-NHEt;

Ac-D-hArg($Et_2$)-Gly-Cys-Phe-D-Trp-Lys-Thr-Cys-Phe-$NH_2$;

Propionyl-D-hArg($Et_2$)-Gly-Cys-Phe-D-Trp-Lys(iPr)-Thr-Cys-Thr-$NH_2$;

Ac-D-Nal(2)-Gly-Cys-Phe-D-Trp-Lys-Thr-Cys-Gly-hArg(Et)$_2$-$NH_2$;

Ac-D-Lys(iPr)-Gly-Cys-Phe-D-Trp-Lys-Thr-Cys-Thr-$NH_2$;

and the like.

More generally, the compounds of the instant invention wherein the peptide is a cyclic hexapeptide are of the formula

X-Cys-D-D-Trp-E-F-Cys-Y (I)

and the pharmaceutically acceptable salts thereof wherein,
X is an N-terminus anchor;
Y is a C-terminus anchor, G-I or alcohol of G-I, e.g. G-I is Thr, threoninol, phenylalaninol, and the like; wherein at least one of X and Y is a cationic anchor;
D is Phe, Tyr, pF-Phe, pCl-Phe;
E is Lys or Lys($R_1$) where $R_1$ is a lower alkyl or fluoroalkyl of from 1 to 8 carbon atoms, especially Lys or Lys(Me);
F is Thr, Val, Ser:
G is D or L configuration of Thr, Phe, or Nal(2); and
I is $-\bar{O}H$, $-\bar{N}HR_2$ where $R_2$ is H or a substituent selected from $R_1$ above.

Additional, more generic examples of the compounds of formula I, which are included as part of this invention are the cyclic hexapeptides bound to the cationic anchor of the invention, including substituted versions thereof, e.g.,

X-Cys-Phe-D-Trp-Lys-Thr-Cys-Thr-$NH_2$;

X-Cys-Phe-D-Trp-Lys-Thr-Cys-Thr-NHEt

X-Cys-Phe-D-Trp-Lys-Thr-Cys-Phe-$NH_2$;

X-Cys-Phe-D-Trp-Lys-Thr-Cys-X;

X-Cys-Phe-D-Trp-Lys-Thr-Cys-Thr-ol;

Ac-D-Phe-Cys-Phe-D-Trp-Lys-Thr-Cys-X;

and the like, where X is the cationic anchor.

Compounds also included within the scope of this invention are the compounds of formula (II), as follows:

X-Cys-Lys-Asn-Phe-D-D-Trp-E-F-Phe-Thr-Ser-Cys-Y (II)

and the pharmaceutically acceptable salts thereof, wherein
X is an N-terminus anchor;
Y is a C-terminus anchor, G-I or alcohol of G-I; wherein at least one of X and Y is a cationic anchor;
D is Phe, Tyr, pF-Phe, pCl-Phe;
E is Lys or Lys($R_1$) where $R_1$ is a lower alkyl or fluoroalkyl of from 1 to 8 carbon atoms;
F is Thr, Val, Ser:
G is Dor L configuration of Thr, Phe, Nal(2), or $(Gly)_m$, and m is 0 to 3; and
I is $-\bar{O}H$, $-\bar{N}HR_2$ where $R_2$ is H or a substituent selected from $R_1$ above.

Examples of the somatostatin-like analogs of formula II derived from somatostatin (cyclical dodecapeptide) bound to the cationic anchor of this invention include the following:

Ac-D-hArg($Et_2$)-Gly-Cys-Lys-Asn-Phe-Phe-D-Trp-Lys-Thr-Phe-Thr-Ser-Cys-OH;

Ac-D-hArg($(CH_2CF_3)_2$)-Gly-Cys-Lys-Asn-Phe-Phe-D-Trp-Lys-Thr-Phe-Thr-Ser-Cys-$NH_2$;

Ac-hArg($(CH_2CF_3)_2$)-Gly-Cys-Lys-Asn-Phe-Phe-D-Trp-Lys-Thr-Phe-Thr-Ser-D-Cys-NHEt; and

Ac-D-hArg($(CH_2CF_3)_2$)-Gly-Cys-D-Phe-Asn-Phe-Phe-D-Trp-Lys-Thr-Phe-Thr-Ser-D-Cys-OH; and

Ac-D-hArg($(CH_2CF_3)_2$)-Gly-Cys-Lys-Asn-Phe-Phe-D-Trp-Lys(Me)-Thr-Phe-Thr-Ser-D-Cys-$NH_2$; and

Ac-D-hArg($Pr_2$)-Cys-D-Lys-Asn-Phe-Phe-D-Trp-Lys-Thr-Phe-Thr-Ser-Cys-OH;

Ac-D-hArg(Me,hexyl)-Gly-Cys-Lys-Asn-Phe-Phe-D-Trp-Lys-Thr-Phe-Thr-Ser-Cys-$NH_2$

More generally, the somatostatin-like cyclic peptide may be either the full dodecapeptide somatostatin ring or ring-substituted analogs thereof bound to the cationic anchor of the invention, for example,

X-Cys-Lys-Asn-Phe-Phe-Trp-Lys-Thr-Phe-Thr-Ser-Cys-OH;

X-Cys-Lys-Asn-Phe-Phe-D-Trp-Lys-Thr-Phe-Thr-Ser-Cys-OH

X-Cys-Lys-Asn-Phe-Phe-D-Trp-Lys-Thr-Phe-Thr-Ser-Cys-$NH_2$;

X-Cys-Lys-Asn-Phe-Phe-D-Trp-Lys-Thr-Phe-Thr-Ser-D-Cys-OH;

X-Cys-Phe-Asn-Phe-Phe-D-Trp-Lys-Thr-Phe-Thr-Ser-Cys-OH

and the like, where X is the cationic anchor.

Many analogs with substitutions within the ring are known and are covered by the scope of this disclosure when the "cationic anchor" defined above is included. Compounds of this invention also include the corresponding reduced ring (i.e., disulfhydryl form).

As discussed, substitutions within the cyclic dodecapeptide and hexapeptide fragments have been reported to yield active analogs. Somatostatin-like analogs having such substitutions are combined with the cationic anchors described herein to provide analogs with unexpectedly superior biological potency and selectivity.

The modifications described herein have applicability to all known classes of somatostatin-like analogs. The data suggests that the cationic anchor of the instant invention acts to facilitate the biological action of the peptide. This disclosure therefore encompasses not only those analogs specifically noted, but all somatostatin analogs having the disclosed cationic anchor on the peptide analog.

In all of the above embodiments, the company may also be prepared as a corresponding pharmaceutically acceptable salt.

## Utility and Assay Procedures

The compounds of this invention are surprisingly potent and long lasting, exhibiting very high potency for inhibition of growth hormone secretion relative to inhibition of the secretion of insulin or glucagon. In particular, the larger compounds, i.e. peptides larger than hexapeptides, show increased inhibition of gastric acid secretion.

A primary measure of somatostatin activity is the ability to moderate the production of insulin, growth hormone, and/or glucagon production in the body.

Bioassays used for the determination of somatostatin effect on hormone regulation in the body are those recognized in the field and include by way of example, measurements of the inhibition of glucose-induced insulin release, insulin-induced glucagon release, and nembutal-or clonidine-induced growth hormone release. A more detailed description of the assays can be found in Examples 4, 5, and 6.

The use of the somatostatin-like analogs of the instant invention includes the treatment of somatostatin-regulated human disease states. Among these are applications in "Carcinoid Syndrome", peptic ulcers, Type II diabetes (Non-Insulin Dependent Diabetes/NIDD), ileostomy-caused diarrhea, suppression of endocrine product secretion from "apudomas" and inappropriate secretion of glucagon and growth hormone in diabetes and modulation of glucose and amino acid uptake from the gastrointestinal tract with particular application to the complications of diabetes including retinopathy, nephropathy, hypertension and coronary artery disease.

The compounds of this invention may be used to cause tumor regression, especially those tumors expressing epidermal growth factor (EGF) receptors, for example, pituitary adenomas, brain tumors (e.g., meningioma) tumors, abdominal (carcinoid) tumors, endocrine tumors and pancreatic tumors. (See, Lamberts, ISI Atlas of Science: Pharmacology, pp. 179-184 (1988) and references cited therein).

Additional clinical applications include, for example, gastrointestinal asociated disease, including upper GI bleeding, gastric and duodenal ulcers, stress gastritis and esophageal varices. Other indications include acromegaly, hemorrhagic pancreatitis and pancreatic fistula, wound healing following abdominal surgery and alleviation of the symptoms of severe trauma and stress due to extreme hyperglycemia.

The compounds of this invention show an increase in the potency and duration of action in vivo compared to known somatostatin analogs. While not wanting to be bound by any theory or explanation of this observation, it is believed that the attachment of the membrane binding anchor to the somatostatin-like cyclic peptide results in a compound having increased membrane affinity which may cause an alteration in tissue partitioning of the analogs. Such properties lead to depoting onto the phospholipid membranes in the body thereby enabling the analogs to be retained within the body for a longer period of time. It is hypothesized that such modifications may also lead to increased receptor affinity by facilitation of the receptor loading in accordance with the proposed model of Schwyzer, supra, and references therein.

## Administration

In the practice of this invention an effective amount of a compound of the invention or a pharmaceutical composition containing same is administered to the subject in need of, or desiring, such treatment. These compounds or compositions may be administered by any of a variety of routes depending upon the specific end use, including orally, parenterally (including subcutaneous, intramuscular and intravenous administration), rectally, buccally (including sublingually), transdermally or intranasally. The most suitable route in any given case will depend upon the use, the particular active ingredient, and the subject involved. The compound or composition may also be administered by means of controlled release, depot implant or injectable formulations as described more fully herein.

In general for the uses as described in the instant invention, it is expedient to administer the active ingredient in amounts between about 0.001 and 10.0 µg/kg body weight, most preferably from about 0.01 to 5.0 µg/kg body weight. For human therapy, the active ingredient will be administered preferably in the range of from about 0.01 to about 2.0 µg/kg/day. This administration may be accomplished by a single administration, by distribution over several applications or by slow release in order to achieve the most effective results. When administered as a single dose, administration will most preferably be in the range of from about 0.01 to 0.10 µg/kg.

The exact dose and regimen for administration of these compounds and compositions will necessarily be dependent upon the needs of the individual subject being treated, the type of treatment, and the degree of affliction or need. In general, parenteral administration requires lower dosage than other methods of administration which are more dependent upon absorption.

A further aspect of the present invention relates to pharmaceutical compositions comprising as an active ingredient a compound of the present invention in admixture with a pharmaceutically acceptable, non-toxic carrier. As mentioned above, such compositions may be prepared for use for parenteral (subcutaneous, intramuscular or intravenous) administration particularly in the form of liquid solutions or suspensions; for oral or buccal administration particularly in the form of tablets or capsules; or intranasally particularly in the form of powders, nasal drops or aerosols.

The compositions may conveniently be administered in unit dosage form and may be prepared by any of the methods well-known in the pharmaceutical art, for example as described in Remington's Pharmaceutical Sciences, 17th edition, Mack Publishing Company, Easton, PA., 1985. Formulations for parenteral administration may contain as common excipients sterile water or saline, alkylene glycols such as propylene glycol, polyalkylene glycols such as polyethylene glycol, oils of vegetable origin, hydrogenated naphthalenes and the like. For oral administration, the formulation can be enhanced by the addition of bile salts and also by the addition of acylcarnitines (Am. J. Physiol. 251:332 (1986)). Formulations for nasal administration may be solid and contain as excipients, for example, lactose or dextran, or may be aqueous or oily solutions for administration in the form of nasal drops or metered spray. For buccal administration typical excipients include sugars, calcium stearate, magnesium stearate, pregelatinated starch, and the like.

When formulated for nasal administration the absorption across the nasal mucous membrane is enhanced by surfactant acids, such as for example, glycocholic acid, cholic acid, taurocholic acid, ethocholic acid, desoxycholic acid, chenodesoxycholic acid, dehydrocholic acid, glycodeoxy-cholic acid, and the like. (See, B.H.Vickery, "LHRH and Its Analogs-Contraception and Therapeutic Applications", Pt. 2, B.H. Vickery and J.J. Nestor, Eds., MTP Press, Lancaster, UK, 1987).

One or more surfactant acids or salts, but preferably a single pharmaceutically acceptable acid salt, can be added to the compounds of the instant invention. Suitable pharmaceutically acceptable surfactant salts

will be those salts which retain the phenomenon of enhanced peptide absorption, as well as the compound's surfactant characteristics, and which are not deleterious to the subject or otherwise contraindicated. Such salts are for example those salts derived from inorganic bases which include sodium, potassium, lithium, ammonium, calcium, magnesium, ferrous, zinc, copper, manganous, aluminum, ferric, manganic salts and the like. Particularly preferred are the ammonium, potassium, sodium, calcium and magnesium salts. Salts derived from pharmaceutically acceptable organic non-toxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-dimethylaminoethanol, 2-diethylaminoethanol, tromethamine, dicyclohexylamine, lysine, arginine, histidine, caffeine, procaine, hydrabamine, choline, betaine, ethylenediamine, glucosamine, methylglucamine, theobromine, purines, piperazine, piperidine, N-ethylpiperidine, polyamine resins and the like. Particularly preferred organic non-toxic bases are isopropylamine, diethylamine, ethanolamine, trimethamine, dicyclohexylamine, choline and caffeine.

The amount of surfactant used for the practice of this invention will be some amount which increases the absorption of the somatostatin analogs over that of other surfactants which also may enhance peptide absorption to a certain degree. It has been found that such an amount is often in the range between 0.2 and 15%, more often 0.2 to 5 percent by weight/volume of the solution. It is preferred that the surfactant be present in an amount between about 0.5 to 4 percent by weight volume, conveniently about 1 percent by weight volume, preferably about 2 percent by weight volume.

It is desirable to deliver the compounds of the present invention to the subject over prolonged periods of time, for example, for periods of one week to one year from a single administration. Various slow release, depot implant or injectable dosage forms may be utilized. For example, a dosage form may contain a pharmaceutically acceptable non-toxic salt of the compound which has a low degree of solubility in body fluids, for example, (a) an acid addition salt with a polybasic acid such as phosphoric acid, sulfuric acid, citric acid, tartaric acid, tannic acid, pamoic acid, alginic acid, polyglutamic acid, naphthalene mono- or disulfonic acids, polygalacturonic acid, and the like; (b) a salt with a polyvalent metal cation such as zinc, calcium, bismuth, barium, magnesium, aluminum, copper, cobalt, nickel, cadmium and the like, or with an organic cation formed from e.g., N,N′-dibenzylethylenediamine or ethylenediamine; or (c) combinations of (a) and (b) e.g. a zinc tannate salt. Additionally, the compounds of the present invention or, preferably, a relatively insoluble salt such as those just described, may be formulated in a gel, for example, an aluminum monostearate gel with, e.g. sesame oil, suitable for injection. Particularly preferred salts are zinc salts, zinc tannate salts, pamoate salts, and the like. Another type of slow release depot formulation for injection or implantation would contain the compound or salt dispersed or encapsulated in a slowly degrading, non-toxic, non-antigenic polymer such as a polylactic acid/polyglycolic acid polymer. The compounds or, preferably, relatively insoluble salts such as those described above may also be formulated in cholesterol matrix pellets, or silastomer matrix implants, particularly for use in animals. Additional slow release, depot implant or injectable formulations, e.g. liposomes, are well known in the literature. See, for example, Sustained and Controlled Release Drug Delivery Systems, J. R. Robinson ed., Marcel Dekker, Inc., New York, 1978.

## Synthesis of the Peptides

The compounds of the instant invention may be synthesized by any techniques that are known to those skilled in the peptide art. An excellent summary of the many techniques so available may be found in J.M. Stewart and J.D. Young, Solid Phase Peptide Synthesis 2nd edit. Pierce Chemical Co., Rockford, Illinois, 1984, and J. Meienhofer, Hormonal Proteins and Peptides, Vol. 2, p. 46., Academic Press (New York), 1973 for solid phase peptide synthesis and E. Schroder and K. Lubke, The Peptides, Vol. 1, Academic Press (New York), 1965 for classical solution synthesis.

In general, these methods involve the sequential addition of one or more amino acids or suitably protected amino acids to a growing peptide chain. Normally, either the amino or carboxyl group of the first amino acid is protected by a suitable protecting group. The protected or derivatized amino acid can then be either attached to an inert solid support or utilized in solution by adding the next amino acid in the sequence having the complimentary (amino or carboxyl) group suitably protected, under conditions suitable for forming the amide linkage. The protecting group is then removed from this newly added amino acid residue and the next amino acid (suitably protected) is then added, and so forth. After all the desired amino acids have been linked in the proper sequence, any remaining protecting groups (and any solid support) are removed sequentially or concurrently, to afford the crude, reduced form of the polypeptide. The peptide

is oxidatively cyclized in dilute aqueous solution using, for example, air ($O_2$), potassium ferricyanide, $I_2$, $ICH_2CH_2I$, and the like, especially potassium ferricyanide. Alternatively, the formation of the disulfide bond may be carried out by oxidation of the peptide chain while the peptide is still on the resin (after cleavage of the thiol protecting groups, if required). Finally the peptide is desalted and purified chromatographically to yield the final product.

The non-naturally occurring amino acids described herein are prepared by methods well known to those skilled in the art and may be used in either solution phase or solid phase peptide synthesis procedures.

Preferred Embodiments of Synthesis

A preferred method of preparing compounds of the present invention involves solid phase peptide synthesis.

In this preferred method the α-amino function of the amino acids is protected by an acid-or base-sensitive group. Such protecting groups should have the properties of being stable to the conditions of peptide linkage formation, while being readily removable without destruction of the growing peptide chain or racemization of any of the chiral centers contained therein. Suitable protecting groups are t-butyloxycarbonyl (Boc), benzyloxycarbonyl (Cbz), o-chloro-benzyloxycarbonyl (Cl-Z), biphenylisopropyloxycarbonyl, t-amyloxycarbonyl, isobornyloxycarbonyl, α,α-dimethyl-3,5-dimethoxybenzyloxycarbonyl, o-nitrophenylsulfenyl, 2-cyano-t-butyloxycarbonyl, 9-fluorenylmethyloxycarbonyl (Fmoc) and the like, especially t-butyloxycarbonyl (Boc).

Particularly preferred side chain protecting groups are, for lysine: Cbz, Fmoc, p-toluenesulfonyl, Boc and adamantyloxycarbonyl; for tyrosine: benzyl (Bzl), o-bromobenzyloxycarbonyl, 2,6-dichlorobenzyl, isopropyl, cyclohexyl, cyclopentyl and acetyl; for serine or threonine: benzyl and tetrahydropyranyl; for cysteine: 4-methoxybenzyl (Bzl-OMe), 4-methylbenzyl, benzyl, acetamidomethyl, ethylcarbamoyl, S-sulfonate; for tryptophan: $N^{IN}$-formyl or no protection.

The C-terminal amino acid is attached to a suitable solid support. Suitable solid supports useful for the above synthesis are those materials which are inert to the reagents and reaction conditions of the stepwise condensation-deprotection reactions, as well as being insoluble in the media used. Suitable solid supports are chloromethylpolystyrene-divinylbenzene polymer, hydroxymethyl-polystyrene-divinylbenzene polymer, and the like, especially chloromethyl-polystyrene-1% divinylbenzene polymer. When the C-terminus of the compound is an amide (where Y of formula (I) is $G\text{-}NHR_2$), a particularly useful support is the p-methylbenzhydrylamino- polystyrene-divinylbenzene polymer described by Rivaille, et al, Helv. Chim. Acta., 54:2772 (1971). The attachment to the chloromethyl polystyrene-divinylbenzene type of resin is made by means of the reaction of the $N^{\alpha}$-protected amino acid, especially the Boc-amino acid, as its cesium, tetramethylammonium, triethylammonium, 1,5-diazabicyclo[5.4.0]undec-5-ene, or similar salt in ethanol, acetonitrile, N,N-dimethylformamide (DMF), and the like, especially the cesium salt in DMF, with the chloromethyl resin at an elevated temperature, for example between about 40 and 60°C, preferably about 50°C, for from about 12 to 48 hours, preferably about 24 hours. The $N^{\alpha}$-Boc-amino acid is attached to the benzhydrylamine resin, for example, by means of an N,N′-diisopropylcarbodiimide (DIC)/1-hydroxybenzotriazole (HBT) mediated coupling for from about 2 to about 24 hours, preferably about 2 hours at a temperature of between about 10 and 50°C, preferably 25°C in a solvent such as dichloromethane or DMF, preferably dichlormethane. The coupling of successive protected amino acids can be carried out in an automatic polypeptide synthesizer as is well known in the art. The removal of the $N^{\alpha}$-protecting groups may be performed in the presence of, for example, a solution of trifluoroacetic acid in methylene chloride, hydrogen chloride in dioxane, hydrogen chloride in acetic acid, hydrogen chloride in i-PrOH, or other strong acid solution, preferably 50% trifluoroacetic acid (TFA) in dichloromethane at about ambient temperature. Following neutralization with triethylamine or similar base, each protected amino acid is preferably introduced in approximately 2.5 molar excess and the coupling may be carried out in dichloromethane, dichloromethane/DMF mixtures, DMF and the like, especially in methylene chloride at about ambient temperature. The coupling agent is normally N,N′-di-cyclohexylcarbodiimide (DCC) in dichloromethane but may be N,N′-di-iso-propylcarbodiimide (DIC) or other carbodiimides either alone or in the presence of HBT, N-hydroxysuccinimide, other N-hydroxyimides or oximes. Alternately, protected amino acid active esters (e.g. p-nitrophenyl, pentafluorophenyl and the like) or symmetrical anhydrides may be used.

At the end of the solid phase synthesis the fully protected polypeptide is removed from the resin. When the linkage to the resin support is of the benzyl ester type, cleavage is by means of aminolysis with an alkylamine or fluoroalkylamine for peptides with a alkylamide C-terminus, or by aminolysis with, for example, ammonia/methanol or ammonia/ethanol for peptides with an unsubstituted amide C-terminus at a

temperature between about -10 and 50° C, preferably about 25° C, for between about 12 and 24 hours preferably about 18 hours. Peptides with a free -COOH carboxy-terminus (C-terminus) may be obtained by HF or other strongly acidic deprotection regime; or by saponification. Alternatively, the peptide may be removed from the resin by transesterification, e.g., with methanol, followed by aminolysis, saponification, or reduction to the alcohol C-terminus with $LiBH_4$. In yet another alternative regime, the peptide C-terminus may be directly reduced to the alcohol while on the resin. The protected peptide may be purified at this point by silica gel chromatography. The removal of the side chain protecting groups from the polypeptide is performed by treating the aminolysis product with, for example, anhydrous liquid hydrogen fluoride in the presence of anisole or other carbonium scavenger, treatment with hydrogen fluoride/pyridine complex, treatment with tris(trifluoroacetyl)boron and trifluoroacetic acid, by reduction with hydrogen and palladium on carbon or polyvinylpyrrolidone, or by reduction with sodium in liquid ammonia, preferably with liquid hydrogen fluoride, and anisole at a temperature between about -10 and +10° C, preferably about 0° C, for between about 15 minutes and 2 hours, preferably about 1 hour. For peptides on the benzyhydrylamine resin, the resin cleavage and deprotection steps may be combined in a single step utilizing liquid hydrogen fluoride and anisole as described above. The fully deprotected polypeptide is then oxidatively cyclized by dissolution in a dilute aqueous or mixed aqueous/organic solvent mixture and treatment with air ($O_2$), potassium ferricyanide, $I_2$, $ICH_2CH_2I$, and the like, especially one equivalent of potassium ferricyanide. The solution is desalted (e.g. BioRad AG-3 anion exchange resin) and purified by a sequence of chromatographic steps employing any or all of the following types: ion exchange on a weakly basic resin in the acetate form; hydrophobic adsorption chromatography on underivatized polystyrene-divinylbenzene (for example Amberlite XAD); silica gel adsorption chromatography; ion exchange chromatography on carboxymethylcellulose; partition chromatography, e.g., on Sephadex G-25, or countercurrent distribution; high performance liquid chromatography (HPLC), especially reverse phase HPLC on octyl- or octadecylsilyl-silica bonded phase column packing.

The preparation of peptides having a C-terminal alcohol function is preferably done using $LiBH_4$ reduction of the corresponding methyl ester or peptide resin ester, prepared as described above.

Thus, another aspect of the present invention relates to a method for preparing compounds of the invention and of the pharmaceutically acceptable salts thereof which process comprises:

removing protecting groups and, optionally, covalently bound solid support from a protected polypeptide to afford a compound of the formulae (I) or (II) disclosed above or a pharmaceutically acceptable salt thereof, wherein the compound comprises:

(a) a cyclical somatostatin-like peptide, and

(b) a cationic anchor attached to one or both of the Cys residues of the peptide.

## Examples

The examples which follow are illustrative and not limiting of the invention. General examples of the synthetic routes to the synthesis of the hArg($R_2$) class of non-naturally occurring amino acids are given in Nestor, et.al., J. Med.Chem. 31:65 (1988) and U.S. Patent 4,667,014 issued May 19, 1987.

The compounds of formula (I) and (II) are synthesized using the following compound, the preparation of which follows. $N^{\alpha}$-t-butyloxycarbonyl-$N^G$,$N^G$ bis-1,1,1-trifluoroethyl-D-homoarginine hydrochloride is prepared as follows:

A mixture of 7.33g of benzyl-$N_{\alpha}$-benzyloxycarbonyl-D-lysinate toluenesulfonate (B. Bezus and L. Zervas, J. Am. Chem. Soc. 83:719 (1961)) and 3.60g. 1,1,1 trifluorodiethylthiourea (M. Uher and J. Jendrichovsky, Coll. Czech 38:289 (1973)) in 50 ml $CH_3CN$ and 50 ml THF was treated with 2.06g $HgCl_2$ and 3.3 g triethylamine. The reaction mixture was heated to 80-90° C for 8 hours followed by the addition of 20% xs of $HgCl_2$, triethylamine, and the thiourea. Heating was continued for 15 more hours. The reaction was cooled to room temperature, filtered through celite and concentrated in vacuo to dryness. The residue was loaded on a silica gel column and eluted with a gradient from $CH_2Cl_2$/MeOH (19:1) to $CH_2Cl_2$/MeOH (9:1) and then from $CH_2Cl_2$/MeOH (9:1) to $CH_2Cl_2$/MeOH (4:1). The fractions containing product were detected by thin layer chromatography (TLC), pooled, and concentrated to dryness to yield 7.6g of yellow foam. The foam was repurified on a second silica gel column eluted with a gradient from $CH_2Cl_2$/MeOH (9:1) to $CH_2Cl_2$/MeOH (4:1) and then isocratic $CH_2Cl_2$(MeOH (4:1). The fractions containing product were detected by TLC, pooled, and concentrated to dryness to yield 7.0g of benzyl $N_{\alpha}$-benzyloxycarbonyl-N $N^G$ bis 2,2,2-trifluoroethyl D-homoarginate toluenesulfonate $[a]_D^{25}$ 10.2° (c 1.5, MeOH).

A 6g portion of the above-named product and 1g 10% Pd/C in 150 ml EtOH was treated with hydrogen

gas at atmospheric pressure for 3 hours. An additional 0.4g 10% Pd/C was added and hydrogenation was continued for 3 more hours. The reaction mixture was filtered through celite, concentrated to dryness to give 4g $N^G$,$N^G$ bis-(2, 2,2-trifluoroethyl)-D-homoarginine toluenesulfonate $[\alpha]_D^{25}$ -7.76° (CO.4, MeOH).

A solution of this compound (1.96g.) in 8 ml 1N NaOH and 8 ml dioxane was treated with 160 mgs MgO and 1.05g di-t-butyldicarbonate at 0°C. The reaction mixture was stirred at 0°C for 1 hour and then at room temperature for 3 hours. The magnesium salt was filtered and the filtrate concentrated under vacuum. The basic solution was washed with anhydrous diethyl ether, then acidified at 0°C with 1N HCl to pH 3.5. The product was extracted from the acidic aqueous solution with ethyl acetate and dried over magnesium sulfate. The drying agent was filtered and the filtrate concentrated to dryness to yield a white foam. The foam was treated with Ag-3 $Cl^-$ beads to convert the product to the chloride salt form. 1.4g of $N^\alpha$-t-butyloxycarbonyl-$N^G$,$N^G$ bis-2,2,2-trifluoroethyl-D-homoarginine hydrochloride, $[\alpha]_D^{25}$ -2.19° (CO.5, MeOH) was isolated.

Example 1: Synthesis of compounds of Formula (I)

In the reaction vessel of a Beckman 990 Peptide Synthesizer was placed 11.11g (4 mmol) of 4-methylbenzhydrylamine resin. Amino acids were sequentially added to the resin by means of a synthesis program. A typical synthesis program is as described in U.S. Patent 4,667,014 issued May 19, 1987, column 21. Similar synthesis programs for use on other commercially available Peptide Synthesizers may also be used.

A. For preparation of a preferred compound of formula (I), the resin was coupled sequentially with a 2.0 to 2.5 molar excess of each protected amino acid and DCC. The resin was treated during successive coupling cycles with

3.09g Boc-Thr(Bzl)-OH and 4g HBT
3.42g Boc-Cys(Bzl-OMe)-OH
3.09g Boc-Thr(Bzl)-OH
4.14g Boc-Lys(Cl-Z)
3.04g Boc-D-Trp-OH

After the coupling of D-Trp, 0.5% indole was added to the deblocking solution. The resin was then treated successively with

2.65g Boc-PheOH
3.42g Boc-Cys(Bzl-OMe)-OH
1.75g Boc-Gly-OH

At this point, the resulting hepapeptide resin was divided into smaller batches. A 3.37g (.75 mmol) batch was carried forward by further coupling in successive cycles with 1g. Boc-D-hArg($CH_2CF_3$)$_2$-OH + 1g HBT followed by 2 ml acetic anhydride in 12.4 ml DMF.

The resin was removed from the reaction vessel, washed with $CH_2Cl_2$, and dried in vacuo to yield 3.07g of protected polypeptide resin. The peptide was deprotected and removed from the resin by treatment with 30ml anhydrous liquid HF in the presence of 3 ml. of anisole (scavenger) in a Kel-F reaction vessel at 0°C for 1 hour. The HF was evaporated under vacuum and the residue of N-Ac-DhArg($CH_2CF_3$)$_2$-Gly-Cys-Phe-DTrp-Lys-Thr-Cys-Thr-$NH_2$, as its HF salt, was washed with ether. The residue was then extracted with nanopure water (~11). The extracts were diluted immediately to a total volume of 41. with nanopure water. The pH of the solution was adjusted to 7.5-8 with dilute $NH_4OH$ solution. The peptide was cyclized by the slow addition of 80 ml of .01M $K_3$ $Fe(CN)_6$ solution. The dilute peptide solution was acidified to pH 4.5 with 10% acetic acid solution and loaded onto a protonated Biorex 70 column (Biorad Laboratories, Hercules, CA.) packed in water. After loading, the remaining salts were washed off the column with 2 liters of water. The peptide was eluted from the column with a gradient from 11. of 5% HOAc to 11. of 50% HOAc and then continuing with 11. of 50% HOAc. 190 fractions (20-30 ml) were collected with an ISCO automated fraction collector. The absorbance at $\lambda = 280$nm on a Hewlett-Packard 8450A UV/VIS spectrophotometer on every fifth fraction was measured. The results were plotted and fractions 30-55, 56-90, and 91-120 were pooled, evaporated to dryness and each was redissolved in 100 ml of water. The crude material was converted to the acetate salt by passage in water through a column of AG3X (a weakly basic tertiary amine resin) which had been converted to the acetate form. Lyophilization of the fractions yielded 250 mgs., 350 mgs. and 125 mgs. respectively. The pool fractions were evaluated by Spectra Physics analytical high performance liquid chromatography (HPLC) system on a Vydac RP-18 column. The 250 mg. + 350 mg. batches were purified by HPLC on a 200g column of Vydac RP-18 (20-30 micron) equilibrated to the running buffer 28% $CH_3CN$/72% $H_2O$ (0.03M in $NH_4OAc$, pH 4.5). The major UV absorbing (280 nm) peak was collected, concentrated to dryness, and lyophilized three times from nanopure water to yield 135 mgs.

of pure cyclized

NAc-D-hArg($CH_2CF_3$)$_2$-Gly-Cys-Phe-DTrp-Lys-Thr-Cys-Thr-$NH_2$, $[\alpha]_D^{25}$=-18.8° (C 0.3, HOAc).

B. Proceeding in a similar manner as set forth in part A, but substituting the appropriate protected amino acids, the following compounds were prepared:

N-Ac-D-hArg($Et_2$)-Gly-Cys-Phe-DTrp-Lys-Thr-Cys-Thr-$NH_2$

N-Ac-D-hArg(Bu)-Gly-Cys-Phe-DTrp-Lys-Thr-Cys-Thr-$NH_2$

N-Ac-D-hArg(methyl,hexyl)-Gly-Cys-Phe-DTrp-Lys-Thr-Cys-Thr-$NH_2$

N-Ac-D-hArg(methyl,butyl)-Gly-Cys-Phe-DTrp-Lys-Thr-Cys-Thr-$NH^2$

N-Ac-D-hArg(Et$_2$)-Gly-Cys-Phe-DTrp-Lys-Thr-Cys-Thr-NH$_2$

N-Ac-D-hArg(Et$_2$)-Gly-Gly-Cys-Phe-DTrp-Lys-Thr-Cys-Thr-NH$_2$

N-Ac-D-hArg(Et$_2$)-Cys-Phe-DTrp-Lys-Thr-Cys-Thr-NH$_2$

N-Ac-L-hArg(Et$_2$)-Cys-Phe-DTrp-Lys-Thr-Cys-Thr-NH$_2$

N-Ac-D-hArg(CH$_2$CF$_3$)$_2$-Cys-Phe-DTrp-Lys-Thr-Cys-Thr-NH$_2$

N-Ac-D-hArg(Et$_2$)-Gly-Cys-Phe-DTrp-Lys-Thr-Cys-Phe-NH$_2$

N-Ac-D-hArg(CH$_2$CF$_3$)$_2$-Gly-Cys-Phe-DTrp-Lys-Thr-Cys-Phe-NH$_2$

N-Ac-D-hArg(CH$_2$CF$_3$)$_2$-Gly-Cys-Phe-DTrp-Lys(Me)-Thr-Cys-Thr-NH$_2$

N-Ac-D-hArg(CH$_2$CF$_3$)$_2$-Gly-Cys-Phe-DTrp-Lys(Me)-Thr-Cys-Phe-NH$_2$

N-Ac-D-hArg(Et$_2$)-Gly-Cys-Tyr-DTrp-Lys-Thr-Cys-Thr-NH$_2$

N-Ac-hArg($Et_2$)-Gly-Cys-Phe-DTrp-Lys-Thr-Cys-Thr-$NH_2$

D-hArg($Et_2$)-Gly-Cys-Phe-DTrp-Lys-Thr-Cys-Thr-$NH_2$

N-Ac-D-hArg($Et_2$)-Gly-Cys-Phe-DTrp-Lys-Thr-Cys-Nal(2)-$NH_2$

N-Ac-D-hArg($CH_2CF_3$)$_2$-D-hArg($CH_2CF_3$)$_2$-Gly-Cys-Phe-DTrp-Lys-Thr-Cys-Phe-$NH_2$

Ac-D-hArg($Et_2$)-D-hArg($Et_2$)-Gly-Cys-Phe-DTrp-Lys-Thr-Cys-Thr-$NH_2$, and the like.

Example 2: Synthesis of compounds of Formula (I) with a C-terminal alkylamide function

The synthesis for compounds having a C-terminal alkylamide function (Y is G-I where I is -$NHR_2$, $R_2$ being alkyl) was changed to allow cleavage from the resin by aminolysis with the appropriate alkylamine. The reaction vessel of Example 1 was loaded with 1.24g of Boc-Thr(Bzl)-0-Resin, prepared by reaction at 50°C in DMF for 24 hr. of 20 mole-percent excess of the dry cesium salt of Boc-Thr(Bzl)-OH with chloromethylpoly-styrene-1%-divinylbenzene (~1mmol available Cl/g resin; BioRad Labs). This yields a protected amino acid linked to the resin through a benzylic ester type of linkage which can later be aminolyzed.

The amino acid-0-resin was coupled sequentially with a 2 to 2.5 molar excess of the appropriate protected amino acid using dispropylcarbodiimide (DIC). Thus the amino acid resin was treated during successive coupling cycles with:

0.857g Boc-Cys(Bzl-0Me)-OH
0.77g Boc-Thr(Bzl)-OH
1.04g Boc-Lys-(Cl-Z)
0.76g Boc-D-Trp-OH, and 0.1% indole was added to the $CF_3CO_2H$ deprotection solution for later deprotections,
0.66g Boc-Phe-OH
0.85g Boc-Cys(Bzl-OMe)-OH
0.44g Boc-Gly-OH
0.99g Boc-D-hArg($Et_2$)-OH and 1g of HBT,
2 ml of acetic anhydride in 8.2 ml of DMF.

The peptide resin was removed from the reaction vessel, washed with $CH_2Cl_2$ and dried in vacuo to yield 2.15g of protected peptide-0-resin. The protected polypeptide was cleaved from the resin by aminolysis with 50 mL. of ethylamine for 8 hours at 0°C. The ethylamine was allowed to evaporate and the resin was extracted with methanol. The dried resin weighed 1.7g and was re-treated with ethylamine to yield an additional 400 mgs of protected peptide. This protected peptide was mixed with 2 ml of anisole and 20ml redistilled (from $CoF_3$) anhydrous liquid HF at 0°C for 1 hour in a Kel-F reaction vessel. The HF was

evaporated under vacuum and the residue was washed with anhydrous diethyl ether. Peptide was dissolved in 4 liters of $H_2O$. The pH of the solution was adjusted to 7.5-8.0 and the compound was oxidatively cyclized with the slow addition of 30 ml of 0.01M $K_3Fe(CN)_6$ solution. The pH was readjusted to 4.5 and the dilute peptide solution was loaded onto protonated BioRex 70 column packed in water. The Fe salts flow through the column. The peptide was eluted from the column with a gradient going from 11.of 5% HOAc to 11 50% HOAc and then continuing with 11.of 50% HOAc. Fractions were pooled and retained for purification based on their optical densitites at $\lambda = 280$nm and analytical RP-HPLC evaluation. The crude material was converted to the $AcO^-$ salt by passage in water through a column of anion exchange resin (AG3X, BioRad) which had been converted to the acetate form. Lyophilization of the eluate yielded 175 mgs of crude peptide.

The peptide was purified by preparative RP-HPLC on a 2.5 x 100cm column of Vydac RP-18 (20-30 micron) equilibrated to the running buffer 25% $CH_3CN$/75% $H_2O$ (0.03 M in $NH_4OAc$, pH 4.5). The major UV absorbing (280nm) peak was collected, concentrated to dryness, and lyopholized three times from nanopure water to yield 35 mgs of cyclized product,

N-Ac-D-hArg($Et_2$)-Gly-Cys-Phe-DTrp-Lys-Thr-Cys-Thr-NHEt,

$[\alpha]_D^{25}$ = -14.1° (CO.4,HOAc).

The synthesis of additional analogues with ethylamide termini were prepared similarily.

Example 3: Synthesis of compounds of Formula (I) having a reduced C-terminus.

Compounds with a reduced C-terminus (Y is the alcohol of G-I) were prepared by reduction of the peptide-0-resin ester linkage with $LiBH_4$. The synthesis of the peptide can proceed without side chain protection except for Lys and Cys. Thus a 1g (1 mmol) portion of Boc-Thr-0-resin was treated successively (as described in Example 1) with 0.73g
Boc-Cys(acetamidomethyl)-OH,
0.55g Boc-Thr-OH,
1.17g Boc-Lys(Fmoc)-OH and 1g HBT,
0.76g Boc-D-Trp-OH, after which 0.1% indole was added to the $CF_3CO_2H$ deprotection solution,
0.66g Boc-Phe-OH,
0.73g Boc-Cys(acetamidomethyl)-OH
0.94g Boc-Gly-OH,
0.99g Boc-D-hArg($Et_2$)-OH and 1g HBT,
2 ml of acetic anhydride in 7.2 ml DMF

The partially protected peptide-0-resin was removed from the vessel, washed with $CH_2Cl_2$, and dried in vacuo. The peptide-0-resin was swollen in 75 ml of dry THF and treated with 145 mg of $LiBH_4$ in four portions. After 1 hr at room temp, 2.5 ml AcOH was added dropwise to destroy the excess $LiBH_4$. The reaction mix was filtered, the residue was washed with glacial AcOH and the filtrate was concentrated. The reduced, partially protected peptide alcohol was obtained by lyophilization from glacial AcOH. The Lys (Fmoc) residue was removed by treatment with 20% piperidine in DMF for 30 min. The solution is neutralized (AcOH) and desalted by hydrophobic chromatography on Amberlite XAD-2 (Rohm & Haas, Philadelphia, PA.) with a $H_2O$ to 50% EtOH gradient. The major, UV absorbing ($\lambda$ max 280) peak is deprotected and cyclized by dropwise treatment with a solution of $I_2$ in MeOH until no more consumption of the color.

The crude product was purified by preparative RP-HPLC on a 2.5 x 100 cm column of Vydac RP-18 (20-30 micron) packing (using 30% $CH_3CN$ (0.04 M in $NH_4OAc$, pH 4.5) as eluent. The major UV absorbing peak was pooled, concentrated to dryness and lyophilized 3 times from $H_2O$ to yield

Ac-D-hArg($Et_2$)-Gly-Cys-Phe-D-Trp-Lys-Thr-Cys-Thr-ol.

Example 4 Insulin-induced glucagon assay

After several days of acclimatization, rats were weigh-grouped and used on the succeeding one to three days. The rats were injected subcutaneously with 40 mg/kg pentobarbitol(Nembutal). Approximately 30 minutes after injection, the jugular and the hepatic portal veins were exposed and an injection of 0.1% bovine serum albumin (BSA)-saline vehicle or 1 IU/1ml/kg porcine insulin in the same vehicle was administered into the jugular vein. At precisely 15 minutes after the intravenous (i.v.)injection a 1 ml blood sample was collected from the hepatic portal vein and the rat sacrificed with $CO_2$.

The blood sample was collected into a syringe containing 0.70 mg Na-EDTA and 0.1 mg Aprotinin, a commercially available protease inhibitor, in 0.1 ml 0.9% saline. The blood sample was mixed in the syringe and transferred to a plastic microcontainer serum-separator tube and held in the dark on ice. Plasma was harvested within 6 hours by centrifugation and stored at -20°C. The test material was administered intramuscularly to the animal immediately prior to the i.v. injection of insulin. Plasma samples were analyzed for levels of glucagon using a commercially available radioimmuneassay kit for animal glucagon (Cambridge Medical Technology).

The percent inhibition of glucagon release was calculated for each animal receiving the test compound by multiplying by 100 the mean glucagon level of "positive controls", where "positive controls" is defined as animals receiving i.v. insulin and no test compound, less the glucagon level of test animal divided by the mean glucagon level of "positive controls" less the mean glucagon level of "negative controls", where "negative control" is defined as animals receiving i.v. vehicle and no test compound. The mean percent inhibition was plotted against dose of test compound and the $ED_{50}$ derived. The $ED_{50}$ represents the dose required to produce 50 percent inhibition of insulin-induced glucagon release.

| Results | $ED_{50}$ |
|---|---|
| N-Ac-D-hArg($Et_2$)-Gly-Cys-Phe-DTrp-Lys-Thr-Cys-Thr-$NH_2$ | 0.15 µg/kg |

In the above described assay, the relative potencies of

N-Ac-D-hArg($Et_2$)-Gly-Cys-Phe-DTrp-Lys-Thr-Cys-Thr-$NH_2$

compared to somatostatin and Sandostatin having the formula

D-Phe-Cys-Phe-D-Trp-Lys-Thr-Cys-Thr-OH

were 50X and 4X, respectively.

Example 5: Glucose-induced insulin assay

After several days of acclimatization, rats were weigh-grouped and used on the succeeding one to three days. The rats were injected subcutaneously with 60 mg/kg pentobarbitol(Nembutal). Approximately 40 minutes after injection, the jugular vein was exposed and an injection of 0.1% BSA-saline vehicle or 0.5 g /2 ml/kg glucose in the same vehicle was administered into the jugular vein. At precisely 5 minutes after the i.v. injection a 1 ml blood sample was collected via the orbital sinus and the rat was sacrificed with $CO_2$.

The blood sample was collected into glass tubes containing 72 USP units lithium heparin and held on ice. Plasma was harvested within 2 hours by centrifugation and stored at -20°C. The test material was administered to the animal 10 minutes prior to the i.v. injection of glucose. Plasma samples were analyzed

for levels of insulin using a commercially available radioimmuneassay kit for animal insulin (Cambridge Medical Technology).

The percent inhibition of insulin release was calculated for each animal receiving the test compound by multiplying by 100 the the mean insulin level of "positive controls", where "positive controls" is defined as animals receiving i.v. glucose and no test compound, less the insulin level of test animal divided by the mean insulin level of "positive controls" less the mean insulin level of "negative controls", where "negative control" is defined as animals receiving i.v. vehicle and no test compound. The mean percent inhibition was plotted against dose of test compound and the $ED_{50}$ derived. The $ED_{50}$ representing the dose required to produce 50 percent inhibition of insulin release.

| Results | $ED_{50}$ |
|---|---|
| N-Ac-D-hArg($Et_2$)-Gly-Cys-Phe-DTrp-Lys-Thr-Cys-Thr-$NH_2$ | 20 µg/kg |

In the above described assay, the relative potencies of

N-Ac-D-hArg($Et_2$)-Gly-Cys-Phe-DTrp-Lys-Thr-Cys-Thr-$NH_2$

compared to somatostatin and Sandostatin were 6X and 0.3X, respectively.

Example 6: Nembutal-induced growth hormone assay

After several days of acclimatization, male rats were weight-grouped and used on the succeeding one to three days. The rats were fasted the day prior to use. Each test rat was given an intramuscular (IM) injection of test compound or vehicle (0.1% BSA-saline) into the right rear leg.

Immediately following the IM injection, a subcutaneous injection of Nembutal (60 mg/kg) was given. Thirteen minutes after Nembutal administration, each rat was exposed to 2 minutes of ether anesthesia followed by the collection of a 1 ml blood sample via the ocular orbit. Serum separator tubes were used to prepare serum from the blood samples. The serum samples were stored at -20°C until analysis by radioimmune assay. The mean percent inhibition of growth hormone was determined for the test compounds using the method as described in Examples 4 and 5 and the $ED_{50}$ derived.

| Results | $ED_{50}$ |
|---|---|
| N-Ac-D-hArg($Et_2$)-Gly-Cys-Phe-DTrp-Lys-Thr-Cys-Thr-$NH_2$ | 0.025 µg/kg |

In the above described assay, the relative potencies of

N-Ac-D-hArg($Et_2$)-Gly-Cys-Phe-DTrp-Lys-Thr-Cys-Thr-$NH_2$

compared to somatostatin and Sandostatin were 265X and 3X, respectively.

Relative selectivities for glucagon/insulin and growth hormone/insulin for

N-Ac-D-hArg($Et_2$)-Gly-Cys-Phe-DTrp-Lys-Thr-Cys-Thr-$NH_2$

were 133 and 769, respectively, versus values for Sandostatin of 11 and 103. Increasing the time interval between dosing of

N-Ac-D-hArg($Et_2$)-Gly-Cys-Phe-DTrp-Lys-Thr-Cys-Thr-$NH_2$

and the challenge agents (i.v. glucose, i.v. insulin and sc nembutal) also revealed the analog to be extremely long-lived compared to somatostatin.

Example 7: Toxicity

Two somatostatin analogs

NAc-D-hArg($CH_2CF_3$)$_2$-Gly-Cys-Phe-DTrp-Lys-Thr-Cys-Thr-$NH_2$ and

N-Ac-D-hArg($Et_2$)-Gly-Cys-Phe-DTrp-Lys-Thr-Cys-Thr-$NH_2$

have been administered intramuscularly to the rat at doses up to 2.43mg/kg in the glucose-induced insulin assay, with no observed adverse effects. In the assay, male rats are anesthetized with pentobarbital and given an intravenous injection of glucose. A blood sample is collected 5 minutes after the glucose injection and assayed at a later time for insulin level. The somatostatin analog is administered intramuscularly at 15 minutes, 1 hour, or 2 hours before the blood sampling. Rats are sacrificed following the blood sampling. A total of 24 rats received a dose of 2.43mg/kg of RS-45917-298 and another 24 rats received a dose of 2.43mg/kg of RS-10962-298. For each compound, 8 rats were injected 15 minutes before blood sampling and 16 rats were injected 2 hours before blood sampling. None of the rats showed any adverse reaction to the dose of either compound.

## Claims

1. A compound of the formula:

X-Cys-D-D-Trp-E-F-Cys-Y (I)

and the pharmaceutically acceptable salts thereof wherein,
X is an N-terminus anchor;
Y is a C-terminus anchor, G-I or alcohol of G-I; wherein at least one of X and Y is a cationic anchor;
D is Phe, Tyr, pF-Phe, pCl-Phe;
E is Lys or Lys($R_1$) where $R_1$ is a lower alkyl or fluoroalkyl of from 1 to 8 carbon atoms;
F is Thr, Val, Ser:
G is D or L configuration of Thr, Phe, or Nal(2); and
I is -OH or -$NHR_2$ where $R_2$ is H or a substituent selected from $R_1$ above.

2. The compound of claim 1 wherein X is an $N^{\omega}$-alkyl-basic amino acid radical attached to the Cys moiety either directly or through a spacer.

3. The compound of claim 2 wherein

X is $A\text{-}(B)_m\text{-}(C)_n$

where A is acyl or H;

B is $hArg(R_1,R_2)$, $Arg(R_1,R_2)$ or $Lys(R_1)$, where $R_1$ is lower alkyl or fluoroalkyl of from 1 to 8 carbon atoms and $R_2$ is independently H or $R_1$;

m is an integer from 1 to 3;

C is Gly, Ala, β-Ala; and

n is an integer from 0 to 5.

4. The compound of claims 3 wherein

X is $A\text{-}(B)_m\text{-}(C)_n$

where A is acyl or H;

B is $hArg(R_1,R_2)$, $Arg(R_1,R_2)$ or $Lys(R_1)$, where $R_1$ is lower alkyl or fluoroalkyl of from 1 to 8 carbon atoms and $R_2$ is independently H or $R_1$;

m is an integer from 1 to 3;

C is Gly, Ala, β-Ala or $hArg(R_1,R_2)$; and

n is 0 or 1.

5. The compound of any one of claims 1 to 4 wherein Y is a C-terminus anchor of the formula $A\text{-}(B)_m\text{-}(C)_n$ where A, B, C, m and n are as defined in claim 3.

6. A compound of claim 1 selected from:

X-Cys-Phe-D-Trp-Lys-Thr-Cys-Thr-$NH_2$;

X-Cys-Phe-D-Trp-Lys-Thr-Cys-Phe-$NH_2$;

X-Cys-Phe-D-Trp-Lys-Thr-Cys-Thr-ol;

X-Cys-Phe-D-Trp-Lys-Thr-Cys-Y; and

Ac-D-Phe-Cys-Phe-D-Trp-Lys-Thr-Cys-Y

Ac-D-Nal-Cys-Phe-D-Trp-Lys-Thr-Cys-Y

X-Cys-Phe-D-Trp-Lys-Thr-Cys-Ac-D-Nal(2)$NH_2$

wherein X is a cationic anchor and Y is a C-terminus anchor.

7. A compound of claim 6 selected from:

Ac-hArg(Et$_2$)-Gly-Cys-Phe-D-Trp-Lys-Thr-Cys-Thr-NH$_2$;

Ac-D-hArg(Et$_2$)-Gly-Cys-Phe-D-Trp-Lys-Thr-Cys-Thr-NH$_2$;

Ac-D-hArg(Bu)-Gly-Cys-Phe-D-Trp-Lys-Thr-Cys-Thr-NH$_2$;

Ac-D-hArg(Et$_2$)-Cys-Phe-D-Trp-Lys-Thr-Cys-Thr-NH$_2$;

Ac-L-hArg(Et$_2$)-Cys-Phe-D-Trp-Lys-Thr-Cys-Thr-NH$_2$

Ac-D-hArg(CH$_2$CF$_3$)$_2$-Cys-Phe-D-Trp-Lys-Thr-Cys-Thr-NH$_2$;

Ac-D-hArg(CH$_2$CF$_3$)$_2$-Gly-Cys-Phe-D-Trp-Lys-Thr-Cys-Thr-NH$_2$;

Ac-D-hArg(CH$_2$CF$_3$)$_2$-Gly-Cys-Phe-D-Trp-Lys-Thr-Cys-Phe-NH$_2$.

Ac-D-hArg(CH$_2$CF$_3$)$_2$-Gly-Cys-Phe-D-Trp-Lys-Thr-Cys-Thr-NHEt

Ac-L-hArg(CH$_2$CF$_3$)$_2$-Gly-Cys-Phe-D-Trp-Lys-Thr-Cys-Thr-NH$_2$;

Ac-D-hArg($CH_2CF_3$)$_2$-Gly-Cys-Phe-D-Trp-Lys(Me)-Thr-Cys-Thr-$NH_2$;

Ac-D-hArg($CH_2CF_3$)$_2$-Gly-Cys-Phe-D-Trp-Lys(Me)-Thr-Cys-Thr-NHEt

Ac-hArg($CH_3$,hexyl)-Gly-Cys-Phe-D-Trp-Lys-Thr-Cys-Thr-$NH_2$;

H-hArg(hexyl$_2$)-Gly-Cys-Phe-D-Trp-Lys-Thr-Cys-Thr-$NH_2$;

Ac-D-hArg($Et_2$)-Gly-Cys-Phe-D-Trp-Lys-Thr-Cys-Thr-NHEt;

Ac-D-hArg($Et_2$)-Gly-Cys-Phe-D-Trp-Lys-Thr-Cys-Phe-$NH_2$;

Propionyl-D-hArg($Et_2$)-Gly-Cys-Phe-D-Trp-Lys(iPr)-Thr-Cys-Thr-$NH_2$;

Ac-D-Nal(2)-Gly-Cys-Phe-D-Trp-Lys-Thr-Cys-Gly-hArg(Et)$_2$-$NH_2$;

Ac-D-Lys(iPr)-Gly-Cys-Phe-D-Trp-Lys-Thr-Cys-Thr-$NH_2$;

Ac-D-hArg($CH_2CF_3$)$_2$-D-hArg($CH_2CF_3$)$_2$-Gly-Cys-Phe-D-Trp-Lys-Thr-Cys-Thr-$NH_2$;

N-Ac-D-hArg($CH_2CF_3$)$_2$-D-hArg($CH_2CF_3$)$_2$-Gly-Cys-Phe-DTrp-Lys-Thr-Cys-Phe-$NH_2$; and

N-Ac-D-hArg($Et_2$)-D-hArg($Et_2$)-Gly-Cys-Phe-DTrp-Lys-Thr-Cys-Thr-$NH_2$,

and the pharmaceutically acceptable salts thereof.

8. A compound of the formula:

X-Cys-Lys-Asn-Phe-D-D-Trp-E-F-Phe-Thr-Ser-Cys-Y (II)

and the pharmaceutically acceptable salts thereof, wherein
X is an N-terminus anchor;
Y is a C-terminus anchor, G-I or alcohol of G-I; wherein at least one of X and Y is a cationic anchor;
D is Phe, Tyr, pF-Phe, pCl-Phe;
E is Lys or Lys($R_1$) where $R_1$ is a lower alkyl or fluoroalkyl of from 1 to 8 carbon atoms;
F is Thr, Val, Ser:
G is D or L configuration of Thr, Phe, Nal(2), or $(Gly)_m$, and m is 0 to 3; and
I is $-\bar{O}H$, $-\bar{N}HR_2$ where $R_2$ is H or a substituent selected from $R_1$ above.

9. The compound of claim 8 wherein X is an $N^{\omega}$-alkyl-basic amino acid radical attached to the Cys moiety either directly or through a spacer.

10. The compound of claim 9 wherein
X is $A-(B)_m-(C)_n$
where A is acyl or H;
B is hArg($R_1$,$R_2$), Arg($R_1$,$R_2$) or Lys($R_1$), where $R_1$ is lower alkyl or fluoroalkyl of from 1 to 8 carbon atoms and $R_2$ is independently H or $R_1$;
m is an integer from 1 to 3;
C is Gly, Ala, β-Ala; and
n is an integer from 0 to 5.

11. The compound of claim 10 wherein
X is $A-(B)_m-(C)_n$
where A is acyl or H;
B is hArg($R_1$,$R_2$), Arg($R_1$,$R_2$) or Lys($R_1$), where $R_1$ is lower alkyl or fluoroalkyl of from 1 to 8 carbon atoms and $R_2$ is independently H or $R_1$;
m is an integer from 1 to 3;
C is Gly, Ala, β-Ala; and
n is 0 or 1.

12. The compound of any one of claims 8 to 11 wherein Y is a C-terminus anchor of the formula $A-(B)_m-(C)_n$ where A, B, C, m and n are as defined in claim 10.

13. A compound of claim 8 wherein the dodecapeptide analog is selected from:

X-Cys-Lys-Asn-Phe-Phe-Trp-Lys-Thr-Phe-Thr-Ser-Cys-OH;

X-Cys-Lys-Asn-Phe-Phe-D-Trp-Lys-Thr-Phe-Thr-Ser-Cys-OH;

X-Cys-Lys-Asn-Phe-Phe-D-Trp-Lys-Thr-Phe-Thr-Ser-D-Cys-OH;

and

X-Cys-Phe-Asn-Phe-Phe-D-Trp-Lys-Thr-Phe-Thr-Ser-Cys-OH

wherein X is the cationic anchor.

14. A process for preparing a compound of the formula:

X-Cys-D-D-Trp-E-F-Cys-Y (I)

wherein,

X is an N-terminus anchor;

Y is a C-terminus anchor, G-I or alcohol of G-I; wherein at least one of X and Y is a cationic anchor;

D is Phe, Tyr, pF-Phe, pCl-Phe;

E is Lys or Lys($R_1$) where $R_1$ is a lower alkyl or fluoroalkyl of from 1 to 8 carbon atoms;

F is Thr, Val, Ser:

G is D or L configuration of Thr, Phe, or Nal(2); and

I is -OH or -$NHR_2$ where $R_2$ is H or a substituent selected from $R_1$ above; and pharmaceutically acceptable salts thereof,

which process comprises:

(a) removing protecting groups from a compound of the formula:

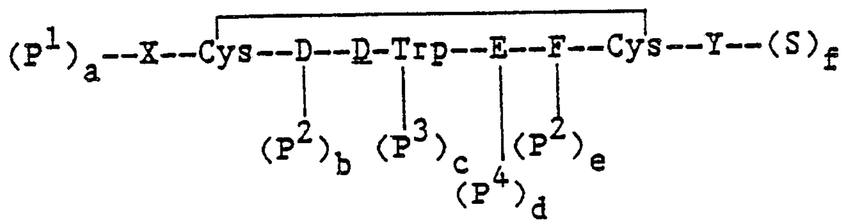

wherein,

X, Y, D, E and F are as defined above;

$P^1$, $P^2$, $P^3$ and $P^4$ are protecting groups wherein $P^1$ is an α-amino protecting group, $P^2$ is a hydroxyl protecting group, $P^3$ is an $N^{IN}$-protecting group and $P^4$ is an ε-amino protecting group;

S is a carboxyl protecting group; and

each of a, b, c, d, e and f is an integer equal to 0 or 1, provided that all of a to f cannot be 0; or

(b) simultaneously removing protecting groups and a polymeric support from a compound of the formula:

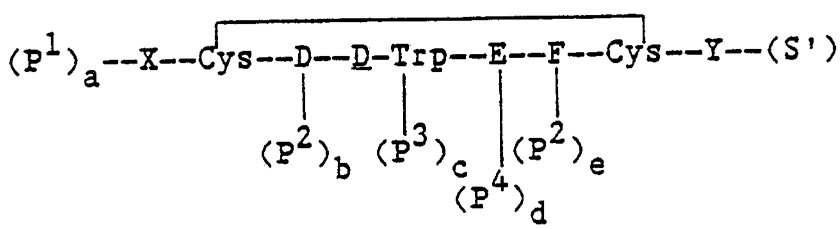

wherein,

X, Y, D, E, F, $P^1$, $P^2$, $P^3$ and $P^4$ are as defined in paragraph (a) above;

each of a, b, c, d and e is an integer equal to 0 or 1, provided that all of a to e cannot be 0; and

S′ is a polymeric support;

or

(c) removing a polymeric support from a compound of the formula:

X--Cys--D--D-Trp--E--F--Cys--Y--(S′)

wherein,

X, Y, D, E, F and S′ are as defined in paragraph (b) above; or

(d) oxidizing a compound of the formula:

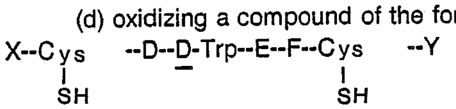

wherein,
X, Y, D, E and F are as defined in paragraph (a) above; or

(d) converting a compound of the formula I to a pharmaceutically acceptable salt; or

(e) converting a salt of the compound of the formula I to another pharmaceutically acceptable salt; or

(f) converting a salt of the compound of the formula I to the free peptide.

15. A process for preparing a compound of the formula:

X-Cys-Lys-Asn-Phe-D-D-Trp-E-F-Phe-Thr-Ser-Cys-Y (II)

wherein
X is an N-terminus anchor;
Y is a C-terminus anchor,G-I or alcohol of G-I; wherein at least one of X and Y is a cationic anchor;
D is Phe, Tyr, pF-Phe, pCl-Phe;
E is Lys or Lys($R_1$) where $R_1$ is a lower alkyl or fluoroalkyl of from 1 to 8 carbon atoms;
F is Thr, Val, Ser:
G is D or L configuration of Thr, Phe, Nal(2), or $(Gly)_m$, and m is 0 to 3; and
I is -OH, -$NHR_2$ where $R_2$ is H or a substituent selected from $R_1$ above, and the pharmaceutically acceptable salts thereof,
which process comprises:

(a) removing protecting groups from a compound of the formula:

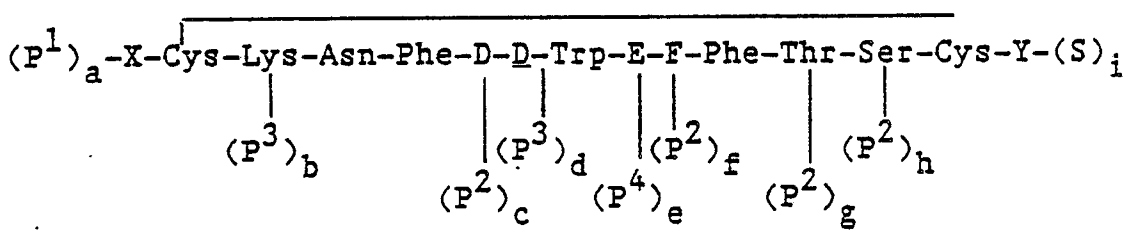

wherein,
X, Y, D, E and F are as defined above;
$P^1$, $P^2$, $P^3$ and $P^4$ are protecting groups wherein $P^1$ is an α-amino protecting group, $P^2$ is a hydroxyl protecting group, $P^3$ is an $N^{IN}$-protecting group and $P^4$ is an ε-amino protecting group;
S is a carboxyl protecting group; and
each of a, b, c, d, e, f, g, h and i is an integer equal to 0 or 1, provided that all of a to i cannot be 0; or

(b) simultaneously removing protecting groups and a polymeric support from a compound of the formula:

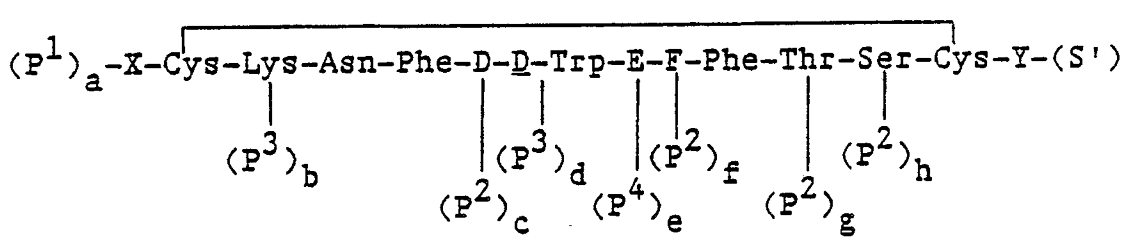

wherein,
X, Y, D, E, F, $P^1$, $P^2$, $P^3$ and $P^4$ are as defined in paragraph (a) above;
each of a, b, c, d, e, f, g and h is an integer equal to 0 or 1, provided that all of a to h cannot be 0; and
S′ is a polymeric support; or

(c) removing a polymeric support from a compound of the formula:

X-Cys-Lys-Asn-Phe-D-D-Trp-E-F-Phe-Thr-Ser-Cys-Y-(S')

wherein,

X, Y, D, E, F and S′ are as defined in paragraph (b) above; or

(d) oxidizing a compound of the formula:

X-Cys(SH)-Lys-Asn-Phe-D-D-Trp-E-F-Phe-Thr-Ser-Cys(SH)-Y

wherein,

X, Y, D, E and F are as defined in paragraph (a) above; or

(d) converting a compound of the formula II to a pharmaceutically acceptable salt; or

(e) converting a salt of the compound of the formula II to another pharmaceutically acceptable salt; or

(f) converting a salt of the compound of the formula II to the free peptide.

16. A compound of any one of claims 1 to 13 for use as a pharmaceutical.

17. A pharmaceutical composition comprising a pharmaceutically acceptable carrier and a compound of any one of claims 1 to 13 and/or a pharmaceutically acceptable salt thereof.

18. A method of preparing a pharmaceutical composition wherein a compound of any one of claims 1 to 13 and/or a pharmaceutically acceptable salt thereof is combined with a pharmaceutically acceptable carrier.

19. The use of a compound of any one of claims 1 to 13 and pharmaceutically acceptable salts thereof in the manufacture of a medicament for treating ulcers, tumors or diabetes.